# EUROPEAN PATENT APPLICATION

(11) **EP 4 767 978 A1**
(43) Date of publication of application: **01.07.2026**
(21) Application number: 24223658.6
(22) Date of filing: 30.12.2024
(51) Int. Cl.: A61B 34/10

(54) **TECHNIQUE FOR DISPLAYING MEDICAL IMAGE DATA INDICATIVE OF A PATIENT ANATOMY HAVING MULTIPLE CONNECTED PARTS**

(71) Applicant: Stryker European Operations Limited, Carrigtwohill, Co. Cork T45 HX08 (IE)
(72) Inventor: KUMAR, Pankaj, 110005 New Delhi (IN); JAIN, Mitesh, 110058 New Delhi (IN); SINGH BEDI, Inderjeet, 110032 Delhi (IN)
(74) Representative: Schott, Jakob Valentin

(57) **Abstract**

A computer-implemented method for displaying medical image data is provided. The method comprises receiving medical image data indicative of a patient anatomy having multiple connected parts that are movable relative to each other, creating at least two different bounding boxes in the medical image data so that each bounding box comprises image data indicative of at least one respective part of the patient anatomy not indicated by the image data of another one of the bounding boxes, and simultaneously displaying images, wherein each of the images is associated with a different one of the at least two different bounding boxes. A computer program, a carrier and a surgical planning system are also provided.

## Description

### Technical Field

The present disclosure generally relates to the field of navigated surgery. In particular, a method for displaying medical image data indicative of a patient anatomy having multiple connected parts that are movable relative to each other is presented. Further, a computer program and a surgical planning system are presented.

### Background

In navigated surgery, relative poses (positions and/or orientations) of a patient anatomy and an instrument used by a surgeon must be detected and tracked. Optically or electromagnetically trackable trackers are commonly used for this purpose. For example, medical image data indicative of positions of radiopaque markers of a tracker can be registered to tracking image data of a camera indicative of positions of optically detectable markers of the tracker or another tracker, wherein the spatial relation between the radiopaque markers and the optically detectable markers is known or determinable. Based on said registration, a model of the tracked instrument may be overlaid over the medical image data and then be visualized on a display as a navigation help for the surgeon.

In common techniques the area comprising the instrument tip and the anatomy to be worked on with the instrument are visualized in detail, allowing the surgeon to accurately navigate the instrument. It is helpful for the area the surgeon is working on to be visualized in detail to reduce a probability for errors during navigation. In general, the more critical the surgery, e.g., a spine surgery or brain surgery, the more detailed (e.g., zoomed-in) the visualization should to be.

However, due to the necessity for a detailed visualization, only a small area / a part of the patient anatomy is typically visualized. Especially in cases where the anatomy to be worked on has multiple connected parts that are movable relative to each other, for example a spine having multiple vertebrae, the surgeon may only be provided with visual information on the part the surgeon is actually working on and may not receive any information on the effects of the surgery on the non-visualized parts of the anatomy that are connected to the visualized part.

As a result, impacts, e.g., detrimental effects, on the non-visualized parts of the patient anatomy may not be known to the surgeon during surgery and damages or at least detrimental effects to the non-visualized anatomy parts may be introduced unknowingly.

### Summary

There is a need for a technique addressing at least some of the aforementioned or other problems. In particular, there is need for a technique informing a surgeon, working on a first anatomy part movably connected to at least one further anatomy part, about the impact of the work on the first anatomy part on the at least one further anatomy.

According to a first aspect, a computer-implemented method for displaying medical image data is provided. The method comprises receiving medical image data indicative of a patient anatomy having multiple connected parts (e.g., anatomical elements, such as bones) that are movable relative to each other, creating at least two different bounding boxes in the medical image data so that each bounding box comprises image data indicative of at least one respective part of the patient anatomy not indicated by the image data of another one of the bounding boxes, and simultaneously displaying images, wherein each of the images is associated with a different one of the at least two different bounding boxes. As a result, the at least two bounding boxes show at least two different anatomical areas allowing a surgeon to assess an impact on the second area due to work performed on the first area.

The medical image data may be or comprise image data generated using a radiation-based imaging technique like computer tomography or x-ray scans, for example.

In some variants, the step of creating the at least two different bounding boxes may comprise selecting a part of the medical image data, and creating at least one of the bounding boxes around the selected part. The selection may be performed automatically or semi-automatically as described in the following.

In some variants, the step of selecting a part of the medical image data may comprise segmenting the medical image data with each segment being indicative of one respective part of the patient anatomy, and selecting one or more image data segments around which the at least one of the bounding boxes is to be created. The segmentation may be based on any known image segmentation technique. The segmentation may be performed at least in part by a trained neural network identifying the respective parts of the patient anatomy. In some variants the segmentation may be based at least in part on user input data defining a seed / origin for at least one segment.

In some variants, the step of creating at least two different bounding boxes may comprise identifying a position of a tracked instrument, and creating at least one of the bounding boxes based on the identified instrument position.

The tracked instrument may be indicated in the received medical image data and the position of the tracked instrument may be identified based at least in part on the medical image data. For example, the position of the tracked instrument may be identified automatically or semi-automatically using known image processing techniques, e.g., identifying the instrument via a trained neural network. In some variants, identifying the position of the tracked instrument may be based at least in part on user input data indicating the instrument in the medical image data. The user input data may form an input for a neural network to facilitating the identification of the position of the tracked instrument and/or increasing an accuracy of the identification of the position of the tracked instrument.

Alternatively or additionally, the medical image data may define an image coordinate system that has been registered with a tracking coordinate system of a tracking system. A registration of the image coordinate system with the tracking coordinate system may have been determined based on any known image registration technique. The method may comprise receiving tracking data indicative of a position of the tracked instrument within the tracking coordinate system and identifying the position of the tracked instrument within the medical image coordinate system based at least in part on the tracking data and the registration between the image coordinate system and the tracking coordinate system.

In some variants, the step of creating one of the bounding boxes based on the identified instrument position may comprise automatically creating a bounding box around the identified position of the tracked instrument. The automatically created bounding box may have a predetermined size around the identified position of the tracked instrument. In other words, at least one of the bounding boxes may be created automatically, e.g., based at least in part on tracking data indicative of a position of a tracked instrument. The at least one automatically created bounding box may have a predefined size and shape. For example, at least one automatically created bounding box may define a rectangular of a predefined size, since common displays for displaying images associated with the created bounding boxes have a rectangular shape. The predefined sized may be based on the size of the patient anatomy within the image data. For example, a size of a movable part of the patient anatomy may be known from, e.g., prior image data and/or parameters of the medical imaging device, or from the image segmentation of the medical image data and the predefined size may be greater than the size of a movable part of the patient anatomy, e.g. two times greater, ensuring that a movable part to be worked on with the tracked instrument is completely enclosed by the automatically created bounding box.

In some variants, at least one of the bounding boxes may be created semi-automatically, e.g., based on a user-input defining at least one of an origin, a size, and a shape for at least one of the bounding boxes.

In some variants, the anatomy may be a spine and each of the anatomy parts may be a vertebra. The method may further comprise, for each bounding box, identifying a spine level of at least one part of the patient anatomy indicated by the image data of the bounding box. In some variants only one spine level may be identified per bounding box. Alternatively, all spine levels indicated in the bounding boxes may be identified. In still further alternatives one or more spine levels may be identified prior to creating a bounding box. For example, all spine levels may indicated in the medical image data may be identified prior to creating the bounding boxes and, optionally, the bounding boxes may be created based at least in part on the identified spine levels.

The spine level identification may be based on an image segmentation of the medical image data. The spine level identification may thus be performed automatically. In some variants the spine level identification may be performed semi-automatically, e.g., based at least in part on user input data. For example, user input data indicating a manually identified spine level may be received and further spine levels may be automatically identified based on the manually identified spine level.

In some variants, the method may further comprise obtaining user input data indicating the at least one part of the patient anatomy for which the spine level is to be identified. Identifying the spine levels only for indicated parts may save computing resources and may reduce the time needed for the (semi-)automatic identification.

In some variants, the method may further comprise displaying the identified spine levels. For example, each of the identified spine levels is displayed adjacent to the corresponding part of the patient anatomy. Displaying the identified spine levels may facilitate analysis of the simultaneously displayed images by a surgeon and may thus improve the quality of a surgery performed by the surgeon.

In some variants, the (simultaneously displayed) images may be displayed in different areas of a single display, e.g., adjacent each other. Additionally or alternatively, at least one of the displayed images at least partly overlaps with another one of the displayed images. For example, a first image may be overlaid over a second image, e.g., as "image in image". In some variants, a first image defined by a bounding box associated with an identified instrument position may be shown on a display and a second image associated with a bounding box independent from the instrument position may be overlaid over the first image, e.g. in a corner thereof.

In some variants, the displayed images may be oriented based on an axis defined based on the medical image data. In other words, the displayed images may be oriented based on the image coordinate system so that a relative orientation between the shown anatomy parts is directly identifiable from the displayed images.

The method may not comprise a surgical step.

According to a second aspect, a computer program is provided. The computer program comprises instructions which, when the program is executed by a processor, cause the processor to carry out the steps of the method according to the first aspect. The computer program may be carried by at least one carrier such as a data stream, a memory or a non-transitory computer-readable storage medium.

According to a third aspect, a surgical planning system is provided. The system comprises at least one processor configured to perform the steps of the method according to the first aspect.

In some variants, the system may further comprise at least one of the following entities: a display configured for displaying the medical image data, a user input device for obtaining user input data, a medical imaging device configured for generating the medical image data, at least one tracker attached to or attachable to at least one of a patient anatomy, a tracked instrument and the medical imaging device, and a tracking system configured to track the at least one tracker.

The display may be any display configured for displaying image data, e.g., a display forming part of or being connected to a computing device, e.g., a control station of the medical imaging device.

The user input device may comprise a common computer keyboard and/or mouse. In some variants the user input device may be a computing device connected to a control station of the medical imaging device.

The medical imaging device may be a common imaging device working on a radiation based technique, e.g., computer tomography or x-ray imaging. The medical imaging device may comprise a common gantry, e.g., a c-arm or an o-arm.

The at least one tracker may be or comprise an optical or electromagnetic tracker. A registration between an image coordinate system and a tracking coordinate system may be determined based at least in part on tracking data indicative of a pose of the at least one tracker.

### Brief Description of the Drawings

Further details, advantages and aspects of the present disclosure will become apparent from the following embodiments taken in conjunction with the drawings, wherein:
- Fig. 1: shows a surgical planning system;
- Fig. 2: shows a flow diagram of a method for displaying medical image data;
- Fig. 3A: shows a display with multiple images indicative of a patient anatomy;
- Fig. 3B: shows the display with two simultaneously displayed images; and
- Fig. 3C: shows another a display with two simultaneously displayed images.

### Detailed Description

In the following description of exemplary embodiments, the same reference numerals are used to denote the same or similar components.

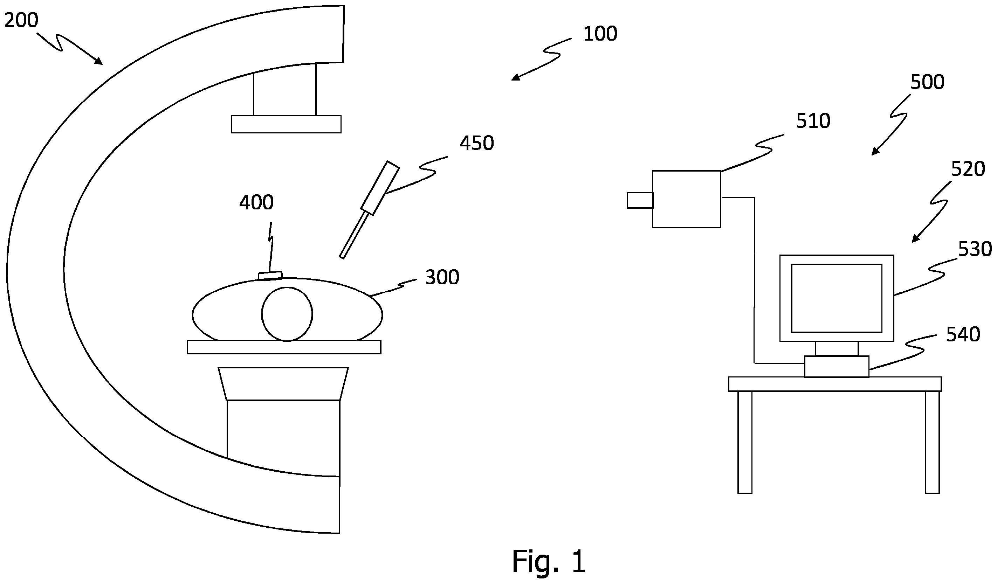

Fig. 1 shows a surgical planning system 100. The shown system 100 comprises a medical imaging device 200 with a c-arm for generating computer tomography images. Other imaging devices and imaging techniques known in the art are contemplated. A patient 300 having an anatomy to be imaged is positioned on a table of the imaging device 200. A patient tracker 400 is attached to the patient and is associated with an image coordinate system. Further, the surgical planning system 100 comprises a trackable surgical instrument 450 that may be used to perform a planned surgery / surgical step on a patient anatomy.

The surgical planning system 100 further comprises a tracking system 500. The tracking system 500 comprises a camera 510 associated with a tracking coordinate system and configured for tracking the patient tracker 400 and/or the surgical instrument 450. Based on the tracking data, the image coordinate system may be registered with the tracking coordinate system and the pose of the surgical instrument 450 may be determined in the image coordinate system and shown in medical image data generated with the medical imaging device 200, e.g., a trajectory or a model of the surgical instrument 450 may be overlaid over the medical image data. Registering the coordinate systems and showing the surgical instrument pose in the medical image data may be performed according to known registration and navigation techniques.

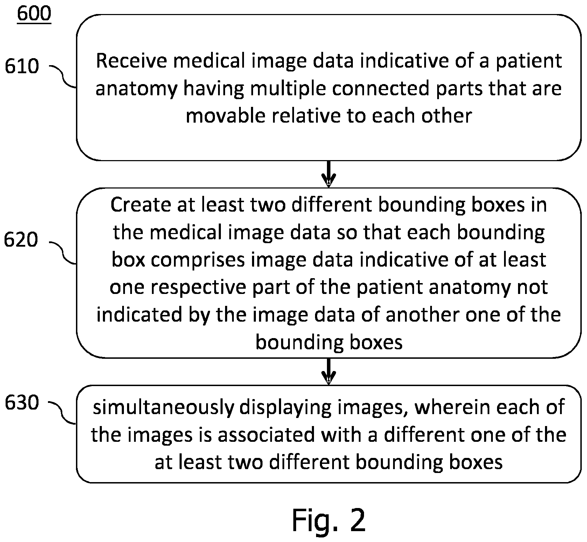

The tracking system 500 further comprises a computing device 520 connected to the camera 510 and to the medical imaging device 200. The computing device 520 comprises a display 530 configured for displaying image data, e.g., the medical image data and, optionally, a pose of the surgical instrument overlaid over the medical image data, and a processor 540 configured to perform a method 600 for displaying medical image data as shown in the flow diagram of Fig. 2, wherein the shown image data is processed to be shown in an improved manner compared to the known techniques navigation techniques as explained with reference to Figs. 2 to 3C below.

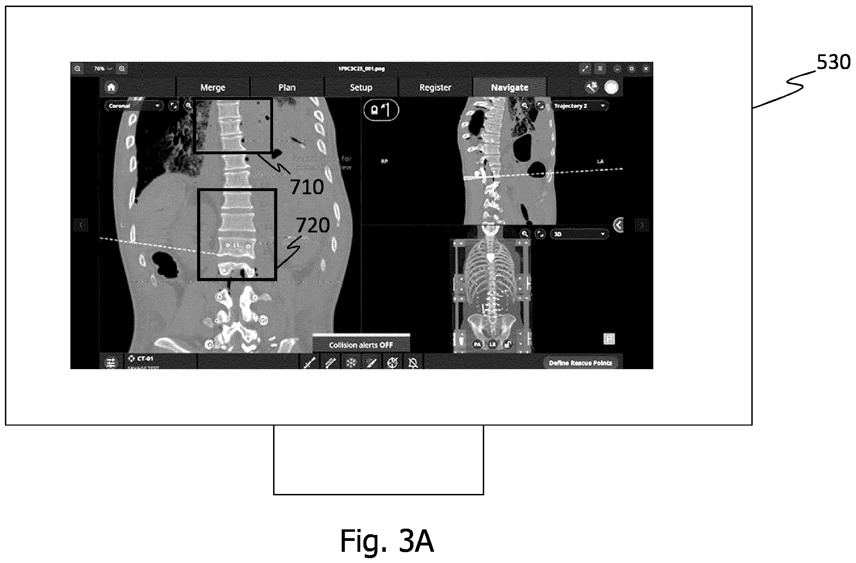

The method 600 shown in Fig. 2 comprises a first step 610 of receiving medical image data. The medical image data has been generated using the medical imaging and is indicative of a patient anatomy having multiple connected parts that are movable relative to each other. Multiple examples of a medical images showing a spine of a patient 300 are shown, e.g., in Fig. 3A.

In detail, Fig. 3A shows the display 530 displaying three images of a spine of a patient 300 simultaneously. Each of the three images shows the spine in a different view. On the left side, the spine is shown in an anteposterior (AP) view. On the top right, the spine is shown in a lateral (LAT) view and on the bottom right a frontal overview of the patient is shown. In each of the anteposterior view and the lateral view a trajectory of a surgical instrument 450 is shown.

Returning to Fig. 2, a second step 620 of the method 600 comprises creating at least two different bounding boxes 710, 720 in the medical image data. Two different bounding boxes 710, 720 are shown in the medical image of Fig. 3A left side. The first bounding box 710 has been created based on user input and comprises vertebrae not directly worked on by a surgeon, i.e., not comprises a trajectory of the surgical instrument 450. The first bounding box 710 may be created by the surgeon using a computer mouse by dragging the mouse while holding a button thereof. Alternatively, the image data may have been segmented using known image segmentation techniques and the user may click on the vertebrae around which a bounding box is to be created. Other methods for creating a bounding box 710, 720 based on user input are contemplated.

The second shown bounding box 720 spaced apart from the first bounding box 710 has been created automatically based on the trajectory of the surgical instrument 450, e.g., to comprise the position of a tip of the instrument 450 and thus comprising the vertebra a surgeon is working on. In other examples, further or other bounding boxes may be created and, optionally, shown in the medical image data. Moreover, any number of bounding boxes 710, 720 may be created automatically or based on user input.

Further, a spine level L1 of the vertebra the surgeon is working is shown, i.e., overlaid over the associated vertebra. The spine level has been automatically identified based on an image segmentation. In some implementations, the spine levels may be identified based on user input instead of automatically, e.g., a surgeon may manually identify a spine level and input the spine level into the computing device 520. In some implementations, the vertebrae for which a spine level is to be automatically identified may be determined based on user input, e.g., a surgeon may click with a computer mouse on displayed, segmented vertebrae for which a spine level is then automatically determined.

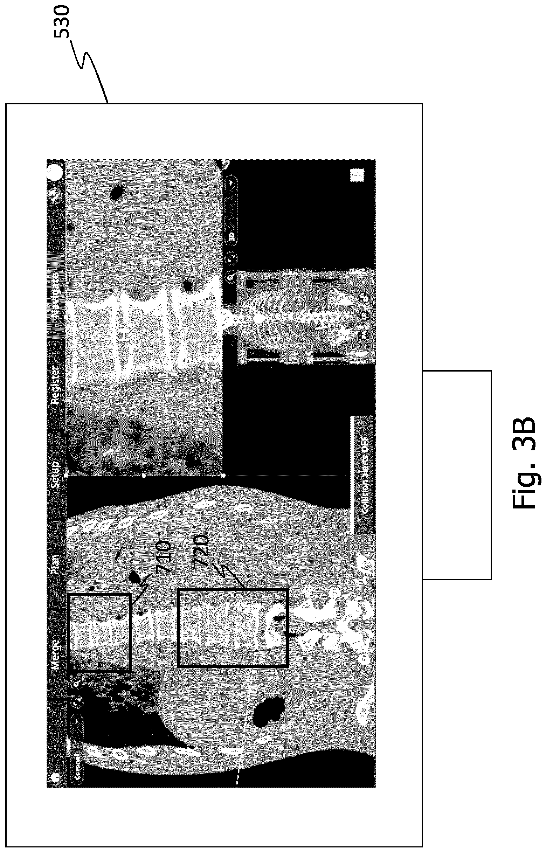

An enlarged view of the first bounding box 710 is shown, e.g., in Fig. 3B top right. Showing the first bounding box in an enlarged view may allow the surgeon to easily assess whether the bounding box 710 has been created and showing the vertebrae as intended by the surgeon. The bounding box 710 may be adapted based on further user input in case the surgeon prefers a different view / bounding box 710.

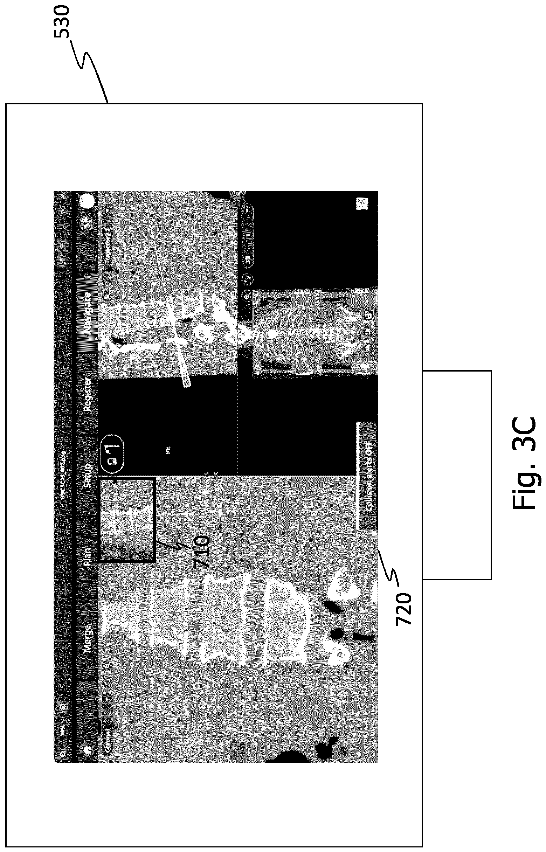

Returning to Fig. 2, a third step 630 of the method 600 comprises simultaneously displaying images, wherein each of the images is associated with a different one of the at least two different bounding boxes 710, 720, e.g., as shown in Fig. 3C left side. In detail, Fig. 3C shows on the left side an enlarged view of the second bounding box 720 with the first bounding box 720 overlaid over the top right corner of the enlarged view of the second bunding box 720. In the shown example, both of the bounding boxes have automatically been oriented based on the medical imaging coordinate system, facilitating the understanding of the shown bounding boxes.

In other implementations the position and or size of the second bounding box 720 may differ from the shown example. Further, the bounding boxes may be shown adjacent instead of being overlaid.

A simultaneous display of the created bounding boxes as shown in Fig. 3C left side allows the surgeon i) a detailed view of the vertebra the surgeon is working on shown in the second bounding box 720 and ii) a simultaneously view of the spaced apart vertebrae comprised by the first bounding box 710. As a result, the surgeon may easily assess the effects on the vertebrae shown in the first bounding box 710 due to his work on the vertebra(e) shown in the second bounding box 720. Thus, the surgeon may be able to base his decisions on a more complete view of the spine of the patient, i.e., on additional information compared to known techniques, so that the overall quality of a navigated surgery may be increased and the risk for a patient may be decreased.

The technique for showing image data as explained with reference to Figs 2 to 3C further allows for an intuitive assessment of the shown data, in particular of an impact of work performed on a first anatomical area on a second anatomical area, and an easy adaption of the shown data according to the preferences of a surgeon.

The features described in relation to the exemplary embodiments shown in the drawings can be readily combined with further features described herein. It is apparent, therefore, that the present disclosure may be varied in many ways. Such variations are not to be regarded as a departure from the scope of the invention as defined by the claims appended hereto.

## Claims

1. A computer-implemented method (600) for displaying medical image data, the method comprising:
receiving (610) medical image data indicative of a patient anatomy having multiple connected parts that are movable relative to each other;
creating (620) at least two different bounding boxes (710, 720) in the medical image data so that each bounding box comprises image data indicative of at least one respective part of the patient anatomy not indicated by the image data of another one of the bounding boxes; and
simultaneously displaying (630) images, wherein each of the images is associated with a different one of the at least two different bounding boxes.

2. The method (600) of claim 1, wherein the step of creating the at least two different bounding boxes (710, 720) comprises:
selecting a part of the medical image data; and
creating at least one of the bounding boxes around the selected part.

3. The method (600) of claim 2, wherein the step of selecting a part of the medical image data comprises:
segmenting the medical image data with each segment being indicative of one respective part of the patient anatomy; and
selecting one or more image data segments around which the at least one of the bounding boxes (710, 720) is to be created.

4. The method (600) of any preceding claim, wherein the step (620) of creating at least two different bounding boxes (710, 720) comprises:
identifying a position of a tracked instrument (450); and
creating at least one of the bounding boxes based on the identified instrument position.

5. The method (600) of claim 4, wherein the tracked instrument (450) is indicated, in particular visualized, in the received medical image data and the position of the tracked instrument is identified based at least in part on the medical image data.

6. The method (600) of claim 4 or 5, wherein the medical image data defines an image coordinate system that has been registered with a tracking coordinate system of a tracking system, further comprising:
receiving tracking data indicative of a position of the tracked instrument (450) within the tracking coordinate system; and
identifying the positioning of the tracked instrument within the medical image coordinate system based at least in part on the tracking data and a registration between the image coordinate system and the tracking coordinate system.

7. The method (600) of any one of claims 4 to 6, wherein the step (620) of creating one of the bounding boxes (710, 720) based on the identified instrument position comprises:
automatically creating a bounding box around the identified position of the tracked instrument (450).

8. The method (600) of claim 7, wherein the automatically created bounding box (710, 720) has a predetermined size around the identified position of the tracked instrument (450).

9. The method (600) of any preceding claim, wherein the anatomy is a spine and each of the anatomy parts is a vertebra.

10. The method (600) of claim 9, further comprising:
for each bounding box (710, 720), identifying a spine level of at least one part of the patient anatomy indicated by the image data of the bounding box.

11. The method (600) of claim 10, wherein the spine level identification is based on an image segmentation of the medical image data.

12. The method (600) of claim 10 or 11, further comprising:
obtaining user input data indicating the at least one part of the patient anatomy for which the spine level is to be identified.

13. The method (600) of any one of claims 10 to 12, further comprising displaying the identified spine levels.

14. The method (600) of claim 13, wherein each of the identified spine levels is displayed adjacent to the corresponding part of the patient anatomy.

15. The method (600) of any preceding claim, wherein the images are displayed in different areas of a single display (530).

16. The method (600) of any of claims 1 to 15, wherein at least one of the displayed images at least partly overlaps with another one of the displayed images.

17. The method (600) of any preceding claim, wherein the displayed images are oriented based on an axis defined based on the medical image data.

18. A computer program comprising instructions which, when the program is executed by a processor (540), cause the processor to carry out the steps of the method (600) of any one of claims 1 to 17, wherein the computer program is optionally carried by at least one carrier such as a data stream, a memory or a non-transitory computer-readable storage medium.

19. A surgical planning system (100) comprising at least one processor (540) configured to perform the steps of the method of any one of claims 1 to 17.

20. The system (100) of claim 19, further comprising at least one of the following entities:
a display (530) configured for displaying the medical image data;
a user input device for obtaining user input data;
a medical imaging device (200) configured for generating the medical image data;
at least one tracker (400) attached to or attachable to at least one of a patient anatomy, a tracked instrument (450) and the medical imaging device;
a tracking system (500) configured to track the at least one tracker.

## Amended claims

### Amended claims in accordance with Rule 137(2) EPC.

1. A computing device (520) for displaying medical image data, comprising:
a processor (540) configured to:
- receive medical image data indicative of a patient anatomy having multiple connected parts that are movable relative to each other;
- create at least a first bounding box (710) in the medical image data and a second bounding box (720) different from the first bounding box (710) in the medical image data so that each of the at least first and second bounding boxes (710, 720) comprises image data indicative of at least one respective part of the patient anatomy not indicated by the image data of another one of the at least first and second bounding boxes (710, 720); and
a display (530) configured to:
- display the medical image data with the at least first and second created bounding boxes (710, 720); and
- simultaneously display at least first and second images, wherein each of the at least first and second images is associated with a different one of the at least first and second bounding boxes (710, 720), wherein the first image is associated with the first bounding box (710) and the second image is associated with the second bounding box (720), and wherein the second image is an enlarged view of the second bounding box (720).

2. The computing device (520) of claim 1, wherein the processor (540) is configured to create at least the first bounding box (710) in the medical image data and the second bounding box (720) different from the first bounding box (710) in the medical image data by:
selecting a part of the medical image data; and
creating at least one of the bounding boxes around the selected part.

3. The computing device (520) of claim 2, wherein the processor (540) is configured to select a part of the medical image data by:
segmenting the medical image data with each segment being indicative of one respective part of the patient anatomy; and
selecting one or more image data segments around which the at least one of the bounding boxes (710, 720) is to be created.

4. The computing device (520) of any preceding claim, wherein the processor (540) is configured to create at least the first bounding box (710) in the medical image data and the second bounding box (720) different from the first bounding box (710) in the medical image data by:
identifying a position of a tracked instrument (450) indicated in the medical image data; and
creating at least one of the bounding boxes based on the identified instrument position.

5. The computing device (520) of claim 4, wherein the tracked instrument (450) is indicated, in particular visualized, in the received medical image data and the position of the tracked instrument is identified based at least in part on the medical image data.

6. The computing device (520) of claim 4 or 5, wherein the medical image data defines an image coordinate system that has been registered with a tracking coordinate system of a tracking system, wherein the processor (540) is configured to:
receive tracking data indicative of a position of the tracked instrument (450) within the tracking coordinate system; and
identify the positioning of the tracked instrument within the medical image coordinate system based at least in part on the tracking data and a registration between the image coordinate system and the tracking coordinate system.

7. The computing device (520) of any one of claims 4 to 6, wherein the processor (540) is configured to create at least the first bounding box (710) in the medical image data and the second bounding box (720) different from the first bounding box (710) in the medical image data by:
automatically creating a bounding box around the identified position of the tracked instrument (450).

8. The computing device (520) of claim 7, wherein the automatically created bounding box (710, 720) has a predetermined size around the identified position of the tracked instrument (450).

9. The computing device (520) of any preceding claim, wherein the anatomy is a spine and each of the anatomy parts is a vertebra.

10. The computing device (520) of claim 9, wherein the processor (540) is configured to:
for each bounding box (710, 720), identify a spine level of at least one part of the patient anatomy indicated by the image data of the bounding box.

11. The computing device (520) of claim 10, wherein the spine level identification is based on an image segmentation of the medical image data.

12. The computing device (520) of claim 10 or 11, wherein the processor (540) is configured to:
obtain user input data indicating the at least one part of the patient anatomy for which the spine level is to be identified.

13. The computing device (520) of any one of claims 10 to 12, wherein the display (530) is configured to display the identified spine levels.

14. The computing device (520) of claim 13, wherein the display unit (540) is configured to display each of the identified spine levels adjacent to the corresponding part of the patient anatomy.

15. The computing device (520) of any preceding claim, wherein the display unit (540) is configured to display the images in different areas of a single display (530).

16. The computing device (520) of any of claims 1 to 15, wherein at least one of the displayed images at least partly overlaps with another one of the displayed images.

17. The computing device (520) of any preceding claim, wherein the displayed images are oriented based on an axis defined based on the medical image data.

18. A computer-implemented method (600) for displaying medical image data performed by the computing device (520) of any of claims 1 to 17.

19. A computer program comprising instructions which, when the program is executed by a computing device (520), cause the computing device (520) to carry out the steps of the method (600) of claim 18, wherein the computer program is optionally carried by at least one carrier such as a data stream, a memory or a non-transitory computer-readable storage medium.

20. A surgical planning system (100) comprising the computing device (520) of any of claims 1 to 17, and, optionally, further comprising at least one of the following entities:
a user input device for obtaining user input data;
a medical imaging device (200) configured for generating the medical image data;
at least one tracker (400) attached to or attachable to at least one of a patient anatomy, a tracked instrument (450) and the medical imaging device;
a tracking system (500) configured to track the at least one tracker.
